# EUROPEAN PATENT APPLICATION

(11) **EP 1 577 294 A1**
(43) Date of publication of application: **21.09.2005**
(21) Application number: 03768425.5
(22) Date of filing: 04.12.2003
(51) Int. Cl.: C07C 335/12, C07D 307/10, C07D 307/38, C07D 409/12, A61K 31/155, A61K 31/381, A61P 25/28

(54) **S-SUBSTITUTED N-1- (HETERO)ARYL ALKYL-N - (HETERO)ARYL ALKYLYSOTHIOCARBAMIDES, METHOD FOR THE PRODUCTION THEREOF, PHYSIOLOGICALLY ACTIVE S-SUBSTITUTED N-1- (HETERO )ARYL ALKYL-N - (HETERO)ARYL ALKYLYSOTHIOCARBAMIDES,A PHARMACEUTICAL COMPOUND AND CURING METHOD**

(30) Priority: 05.12.2002 RU 2002132699
(71) Applicant: Institut Fiziologicheski Aktivnykh Veschestv Rossiyskov Akademii Nauk, Moskovskaya obl., 142432 (RU); MEDIVATION, INC., San Francisco, CA 94107 (US)
(72) Inventor: TKACHENKO, Sergey Evgenievich, Chernogolovka, Moskovskaya obl., 142432 (RU); PROSHIN, Aleksey Nikolaevich, Chernogolovka, Moskovskaya obl. 142432 (RU); BACHURIN, Sergey Olegovich, Chernogolovka, Moskovskaya obl., 142432 (RU); LERMONTOVA, Nadezhda Nikolaevna, Chernogolovka, Moskovskaya obl., 142432 (RU); ZEFIROV, Nikolay Serafimovich, Moscow, 117421 (RU); DMITRIEV, Maksim Eduardovich, Chernogolovka, Moskovskaya obl., 142432 (RU); GRIGORIEV, Vladimir Viktorovich, Chernogolovka, Moskovskaya obl., 142432 (RU)
(74) Representative: Woods, Geoffrey Corlett
(86) International application number: PCT/RU2003/000536
(87) International publication number: WO 2004/050612

(57) **Abstract**

The invention relates to s-substituted N-1-[(hetero)aryl]alkyl-N-1-[(hetero)aryl]alkylysothio-carbamides and the physiologically active salts and bases thereof, embodied in the form of racemic mixtures (or stereoisomer mixture) or individual optical isomers. The specific physiological activity of said compounds makes it possible to use them in the form of molecular tools. Said invention also relates to Pharmaceutical compositions, containing said compounds and to a method for preventing and curing various diseases including neurodegenerative diseases as, for example Alzheimer's disease (AD). The pharmacological effect of said compounds is based on the combined cognitive-stimulating and neuroprotective action thereof, which is produced by acting on chemo-controlled calcium channels of neurone membranes, in particular the neurones regulated by glutamate receptors of the central neural system (CNS).

## Description

This invention relates to new derivatives of N,S,N'(N')-tri(tetra)-substituted isothiocarbamides, which exhibit physiological activity. More specifically, the present invention relates to S-substituted N-1-[(hetero)aryl]alkyl-N'-1-[(hetero)aryl]alkylisothio-carbamides and their physiologically active salts and bases, embodied in the form of racemic mixtures (or mixtures of stereoisomers) or in the form of individual optical isomers; to the specific physiological activity of these compounds, which allows them to be used as "molecular tools"; to pharmaceutical compositions containing said compounds; and to a method for treating and preventing the development of various diseases, including neurodegenerative diseases such as Alzheimer's disease (AD). The pharmacological effect of these compounds is based on their complex cognitive-stimulating and neuroprotective action, which is produced by acting on the chemically controlled calcium channels of nerve cell membranes, in particular those regulated by the glutamate receptors of the central nervous system (CNS).

It has recently been demonstrated that potentiation (or positive modulation) of one of the sub-types of the glutamate receptors, the "amino-3-hydroxy-5-methyl-isoxazole-4-propionic acid/kainic acid" receptors (AMPA/KA receptors) leads to memory enhancement in experimental animals (Yamada K.A. Modulating excitatory synaptic neurotransmission: potential treatment for neurological disease. Neurobiological Dis., 1998, 5, (2), 67-80). In this connection, the search for compounds capable of potentiating AMPA/KA receptors is an unconventional and promising approach to the creation of new drugs for the treatment of AD and other diseases which involve disorders of cognitive functions in man. (Lynch, G. Memory and the brain: unexpected chemistries and a new pharmacology. J. Neurobiol. Learn and Memory, 1998, 70, (1-2), 82-100). In addition, they could be useful in cases requiring substantial enhancement of memory and activation of the learning process, i.e. they could serve as cognitive stimulators.

On the other hand, it is known that a wide range of neurological diseases (such as AD, Huntington's chorea, amyotrophic lateral sclerosis, and also cerebral ischemia) is associated with the excitotoxic action of neuromediator excitatory aminoacids - glutamate and aspartate (Doble A. The Role of Excitotoxicity in Neurodegenerative Disease: Implication for Therapy. Pharmacology and Therapeutics, 1999, **81,** (3), 163-221). In accordance with this mechanism, depolarization of neuronal membranes during the prolonged activation of CNS glutamate receptors, primarily N-methyl-D-aspartate receptors (NMDA receptors) leads to disturbance of calcium homeostasis in the nerve cells and initiates a whole series of pathological processes, resulting in the death of nerve cells (D.W. Choi, Neuron, 1988, 1, 623-634). The search for selective antagonists of cerebral NMDA receptors, capable of protecting nerve cells against the neurotoxic action of excessive concentrations of calcium ions is therefore an extremely promising approach to the creation of new neuroprotectors for the treatment of and protection against a wide range of neurodegenerative diseases (Parsons C.G., Danysz W., Quack G. Glutamate in CNS Disorders as a Target for Drug Development: An Update. Drugs News Perspect., 1998, 11, (9), 523-580).

As a result of research aimed at finding compounds capable of positively modulating AMPA/KA receptors and at the same time possessing anti-NMDA activity, the inventors have discovered an extensive group of S-substituted N-1-[(hetero)aryl]alkyl-N'-1-[(hetero)-aryl]alkylisothiocarbamides which possess a unique combination of said types of activity.

The present invention relates to new S-substituted N-1-[(hetero)aryl]alkyl-N'-1-[(hetero)aryl]alkylisothiocarbamides in the form of free bases and salts with pharmacologically acceptable acids, and also individual optical isomers of said compounds or mixtures of the latter, which all together are represented by general formula (I): 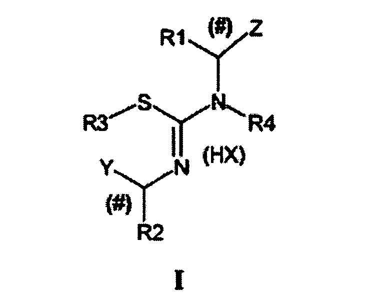 in which:
Y and Z may be the same or different and are, independently, possibly substituted aryl or possibly substituted (possibly partially or fully hydrogenated) heteroaryl;
the symbol (#) signifies the possibility of the presence of a chiral carbon atom;
R¹ and R² may be the same or different and are, independently, H or lower alkyl;
R³ is lower alkyl, possibly substituted alkenyl, possibly substituted cycloalkyl, cycloalkenyl, aralkyl or a group with the general formula:

   -(CH₂)ₙ-W

   in which: n = 1-4, while W is selected from a group consisting of:
   possibly substituted aryl;
   possibly substituted (possibly partially or fully hydrogenated) heteroaryl;
   possibly substituted cycloalkyl;
   ethenyl, optionally mono- or di-substituted with lower alkyl;
   a COOR⁵ group (in which R⁵ is H, lower alkyl, phenyl);
   a CH₂OR⁵ group (in which R⁵ has the values specified above);
   a NR⁶R⁷ group (in which R⁶ and R⁷ may be the same or different, and each is, independently:
      H; alkyl; cycloalkyl; aralkyl;
      one of the substituents R⁶ or R⁷ may be an acyl group C (O) R⁵, in which R⁵ has the values specified above;
      or in which R⁶ and R⁷ may together form an optionally substituted 1,4-butylene or 1,5-pentamethylene chain, and also a -CH₂CH₂-O-CH₂CH₂-chain);
   a N (R⁸)₃⁺X⁻ group (in which R⁸ is lower alkyl, and X is
      the anion of a pharmacologically acceptable acid) an N-phthalimido group, possibly substituted in the benzene ring.
R⁴ is H, lower alkyl, possibly substituted aryl, phenylethyl, possibly substituted in the phenyl ring, [(hetero)aryl]methyl or 1-[(hetero)aryl]ethyl;
   **where:**
   i) **R**^{**4**} **may have the values indicated only when R**^{**2**} **is** **not H;**
   ii) when R² is H, R⁴ is H, lower alkyl, aryl or 2-phenylethyl, possibly substituted in the phenyl ring, in the form of bases or salts of these with pharmacologically acceptable acids HX in the form of a racemic mixture or a mixture of stereoisomers.

The term "lower alkyl" used in the definitions given above and in the following description signifies an alkyl group with a straight or branched chain, containing from 1 to 4 carbon atoms, examples of which are methyl, ethyl, isopropyl, butyl, sec-butyl, tert-butyl and similar.

The term "cycloalkyl" signifies a cyclic saturated hydrocarbon group with 5-7 ring carbon atoms, examples of which include cyclopentyl, cyclohexyl and similar.

The term "cycloalkenyl" signifies a cyclic unsaturated hydrocarbon group with 5-7 ring carbon atoms, examples of which include cyclopentenyl, cyclohexenyl and similar.

The term "aryl" signifies an unsubstituted or substituted phenyl or naphthyl group. Phenyl group substituents may be halogens (such as fluorine, chlorine, and similar), lower alkyl groups (such as methyl, ethyl, iso-propyl and similar), lower alkoxy groups (such as methoxy, ethoxy, iso-propoxy and similar), cyano, nitro, trihalomethyl groups (such as trifluoromethyl and similar), optionally substituted amino groups (such as amino, dimethylamino, acetylamino, N-piperidino, N-phthalimido and similar groups), acyl groups (such as formyl, acetyl, benzoyl and similar), carboxamide groups (such as N,N-diethylcarboxamido and similar groups), carboxy groups, carbalkoxy groups and similar. Naphthyl group substituents may be fluorine, chlorine, bromine, methyl and methoxy groups.

The term "aralkyl" signifies an above-defined aryl to which an above-defined alkyl group has been added.

The term "heteroaryl" signifies a 5- or 6-membered N-, O-, or S-heteroaromatic ring or its benzo-derivative. Examples of appropriate substituents include optionally substituted furan, thiophene, pyrrole, indole, pyridine, quinoline, etc.

The term "halogen" employed here means chlorine, bromine, fluorine or iodine.

The term "alkoxy" signifies an AlkO- group, in which the alkyl fragment is an alkyl group as defined above. Examples of alkoxy groups include methoxy, ethoxy, butoxy and similar groups.

The term "acyl" signifies a COR* group (in which R* has the values H, alkyl, aryl and aralkyl, defined above). Examples of acyl groups include formyl, acetyl, benzoyl, phenylacetyl and similar groups.

The term "pharmacologically acceptable acids" encompasses all pharmacologically acceptable acids, both inorganic (such as hydrochloric, sulfuric, phosphoric, etc.), and also organic (such as oxalic, citric, tartaric, maleic, succinic, methylsulfuric, p-toluenesulfonic, etc.).

Among the compounds of formula (I), which constitute one of the subjects of the present invention, the following three sub-groups of compounds, which can be represented by formulae (I.1), (I.2) and (1.3), given below, are preferred.

I.1. In particular, a first group of preferred compounds comprises: **S-substituted N-[(hetero)aryl]-methyl-N'-1-[(hetero)-aryl]alkylisothiocarbamides** of general formula **I.1:** 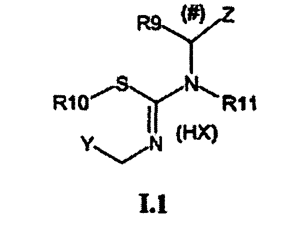 in which:
Y and Z have the values specified above for formula I; the symbol (#) signifies the possibility of the presence of a chiral carbon atom;
R⁹ is H or lower alkyl;
R¹⁰ is lower alkyl, alkenyl, aralkyl or a group with the general formula:

   -(CH₂)ₘ-V

   in which: m = 1-2, and V is selected from the group consisting of:
   **(where m = 1)**
   possibly substituted aryl;
   possibly substituted cycloalkyl;
   ethenyl, optionally monosubstituted with lower alkyl;
   a COOR⁵ group (in which R⁵ has the values specified above);
   a CH₂OR⁵ group (in which R⁵ has the values specified above);
   a diphynylphosphinyl group;
   **(where m = 2)**
   aryl;
   a COOR⁵ group (in which R⁵ has the values specified above);
   N-piperidine;
   N-morpholine;
   N-pyrrolidine;
   an N-phthalimido group, possibly substituted in the benzene ring.
R¹¹ is H, lower alkyl or 2-phenylethyl, possibly substituted in the phenyl ring.

I.1.1. Within the framework of group I.1, the most preferred are **S-substituted N-[(hetero)aryl]methyl-N'-1-(R,S)-phenylethylisothiocarbamides** in the form of a racemic mixture (or an additive mixture of stereoisomers) of general formula **I.1.1:** 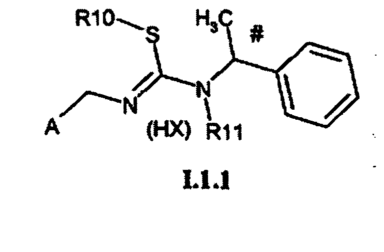 in which:
A is aryl, and also a heterocyclic substituent, namely:
   optionally substituted 2- or 3-furyl,
   optionally substituted 2- or 3-thienyl,
   optionally substituted 2-pyrrolyl,
   optionally substituted 3-indolyl;
   optionally substituted 2-tetrahydrofurfuryl.
R¹⁰ has the values specified above for formula I.1;
R¹¹ has the values specified above for formula I.1;

I.2. A second sub-group of most preferred compounds among the substances corresponding to formula I comprises: **S-substituted N-[(hetero)aryl]-methyl-N'-[(hetero)aryl]-methylisothiocarbamides** of general formula **I.2:** 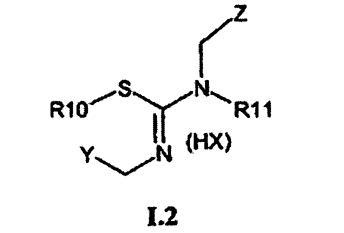 in which:
Y and Z have the values specified above for formula I;
R¹⁰ has the values specified above for formula I.1;
R¹¹ has the values specified above for formula I.1;

1.2.1. Within the framework of group I.2, the most preferred are **S-substituted N'[(hetero)aryl]methyl-N'-(heteroaryl)methylisothiocarbamides** of general formula **I.2.1:** 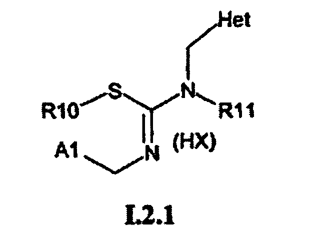 in which:
Het is:
   optionally substituted 2- or 3-furyl;
   optionally substituted 2- or 3-thienyl;
   optionally substituted 2-pyrrolyl;
   optionally substituted 3-indolyl;
   optionally substituted 2- tetrahydrofurfuryl.
A¹ is:
   phenyl, (optionally mono- or di-substituted with such substituents as halogen, lower alkyl, methoxy, ethoxy, trifluoromethyl);
   methylenedioxyphenyl;
   2-furyl;
   2-tetrahydrofurfuryl.
R¹⁰ has the values specified above for formula I.1;
R¹¹ has the values specified above for formula I.1;

Even more preferred in the series of isothiocarbamides of formula I.1.1 are the two groups of compounds I.1.1.1 and 1.1.1.2:
I.1.1.1 **S-substituted N-[(hetero)aryl]methyl-N'-[1-R(+)-phenylethyl]iso-thiocarbamides** of general formula **I.1.1.1:** 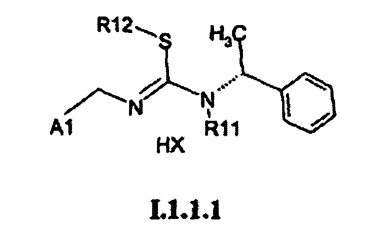 in which:
   A¹ has the values specified above for formula I.2.1.
   R¹¹ has the values specified above for formula I.1;
   R¹² is selected from the group consisting of:
      lower alkyl;
      allyl;
      cyclohexenyl;
      benzyl, optionally substituted in the benzene ring;
      2-phenylethyl;
      2-(ethoxycarbonyl)ethyl;
      2-N-phthalimidoethyl.
I.1.1.2. **S-substituted N-[(hetero)aryl]-methyl-N'-[1-S(-)-phenylethyl]iso-thiocarbamides** of general formula **I.1.1.2:** 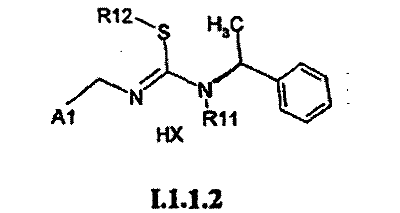 in which:
   A¹ has the values specified above for formula 1.2.1.
   R¹¹ has the values specified above for formula I.1;
   R¹² has the values specified above for formula I.1.1.1

A third sub-group of most preferred compounds among the isothiocarbamides corresponding to formula I is the group of diastereomer compounds 1.3, represented by four subgroups: I.3a, I.3b, I.3c and I.3d:
I.3a. **S,N'-substituted N-[1-S(-)-phenylethyl]-N'-[(1-S(-)-phenylethyl]-isothiocarbamides** of general formula **I.3.a:** 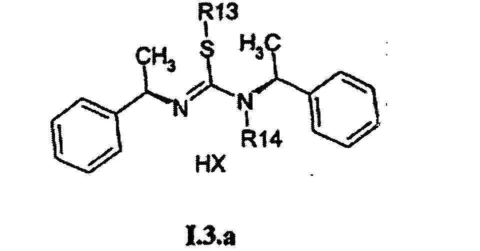 in which:
   R¹³ is selected from the group consisting of:
      lower alkyl;
      allyl;
      cyclohexenyl;
      benzyl, optionally substituted in the benzene ring;
      2-(phenyl)ethyl;
   R¹⁴ is lower alkyl, optionally substituted benzyl, (heteroaryl)methyl or 2-phenylethyl;
I.3b. **S,N'-substituted N-[1-S(-)-phenylethyl]-N'-[1-(R(+)-phenylethyl]-isothiocarbamides** of general formula **I.3.b:** 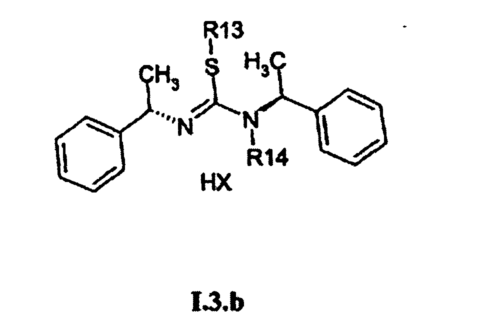 in which:
   R¹³ and R¹⁴ have the values specified above for formula I.3a;
I.3c. **S,N'-substituted N-[1-R(+)-phenylethyl]-N'-[1-(S(-)-phenylethyl]-isothiocarbamides** of general formula **I.3.c:** 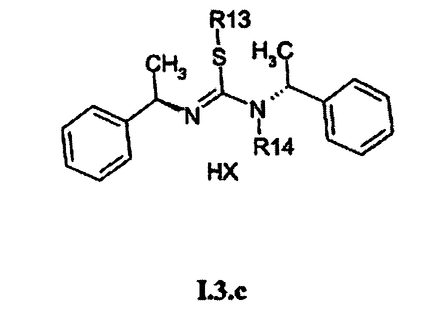 in which:
   R¹³ and R¹⁴ have the values specified above for formula I.3a;
I.3d. **S,N'-substituted N-[1-R(+)-phenylethyl]-N'-[1-(R(+)-phenylethyl]-isothiocarbamides** of general formula **I.3.d:** 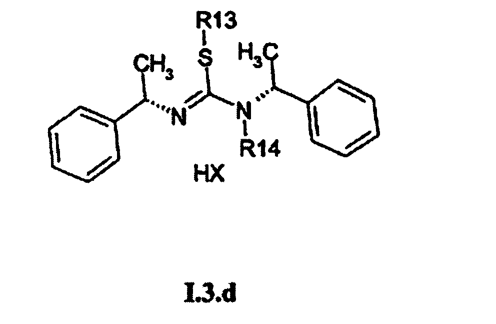 in which:
   R¹³ and R¹⁴ have the values specified above for formula I.3a.

The following compounds of formula **I** (in the form of pharmacologically acceptable salts and/or free bases) are most preferable: S-methyl-N-1-S(-)-phenylethyl-N'-1-S(-)phenylethylisothiocarbamide and S-methyl-N-1-S(-)-phenylethyl-N'-(4-methoxy) benzyl-N'-1-S (-) - phenylethylisothiocarbamide.

The following compounds of formula **I.1** (in the form of pharmacologically acceptable salts and/or free bases) are most preferable: S-methyl-N-4-fluorobenzyl-N'-[1-S(-)-phenylpropyl]-N'-isobutyl-isothiocarbamide and S-methyl-N-4-fluorobenzyl-N'-[1-R(+)-phenylpropyl]-N'-isobutylisothiocarbamide.

The following compound of formula **I.1.1** (in the form of pharmacologically acceptable salts and/or free bases) is most preferable: S-methyl-N-4-methoxybenzyl-N'-3,4-dimethoxyphenetyl-N'-1-phenylethylisothiocarbamide.

The following compounds of formula **I.2** (in the form of pharmacologically acceptable salts and/or free bases) are most preferable:
S-methyl-N-benzyl-N'-....benzylisothiocarbamide, S-ethyl-N-benzyl-N'-benzylisothiocarbamide, S-benzyl-N-benzyl-N'-benzylisothiocarbamide, S-4-nitrobenzyl-N-benzyl-N'-benzylisothiocarbamide, S-4-carboxybenzyl-N-benzyl-N'-benzylisothiocarbamide, S-allyl-N-benzyl-N'-benzyl-isothiocarbamide, S-2-cyclohexenyl-N-benzyl-N'-benzyl-isothiocarbamide, S-2-phenylethyl-N-benzyl-N'-benzyl-isothiocarbamide, S-2-ethoxycarbonyl-ethyl-N-benzyl-N'-benzylisothiocarbamide.

The following compounds of formula **I.2.1.** (in the form of pharmacologically acceptable salts and/or free bases) are most preferable: S-methyl-N-2-tetrahydrofurfuryl-N'-5-methylfurfuryl-N'-2-phenylethyl-isothiocarbamide and S-methyl-N-piperonyl-N'-2-thienylmethyl-N'-isopropylisothiocarbamide.

The following compounds of formula **I.1.1.1** (in the form of pharmacologically acceptable salts and/or free bases) are most preferable: S-methyl-N-benzyl-N'-1-R(+)-phenylethyl-N'-(cyclohex-3-enyl)methylisothiocarbamide and S-methyl-N-benzyl-N'-1-R(+)-phenylethyl-N'-2-phenyl-ethylisothiocarbamide.

The following compounds of formula **I.1.1.2** (in the form of pharmacologically acceptable salts and/or free bases) are most preferable: S-methyl-N-benzyl-N'-1-S(-)-phenylethyl-N'-(cyclohex-3-enyl)methylisothiocarbamide and S-methyl-N-benzyl-N'-1-S(-)-phenylethyl-N'-2-phenyl-ethylisothiocarbamide.

The following compound of formula **I.3.a** (in the form of pharmacologically acceptable salts and/or free bases) is most preferable: S-methyl-N-[1-S(-)-phenylethyl]-N'-[1-S(-)-phenylethyl]-N'-isobutylisothiocarbamide.

The following compound of formula **I.3.b** (in the form of pharmacologically acceptable salts and/or free bases) is most preferable: S-methyl-N-[1-S(-)-phenylethyl]-N'-[1-R(+)-phenylethyl]-N'-isobutylisothiocarbamide.

The following compound of formula **I.3.c** (in the form of pharmacologically acceptable salts and/or free bases) is most preferable: S-methyl-N-[1-R(+)-phenylethyl]-N'-[1-S(-)-phenylethyl]-N'-isobutylisothiocarbamide.

The following compound of formula **I.3.d** (in the form of pharmacologically acceptable salts and/or free bases) is most preferable: S-methyl-N-[1-R(+)-phenylethyl]-N'-[1-R(+)-phenylethyl]-N'-isobutylisothiocarbamide.

Compounds of formula **I** according to the invention are prepared using methods known in this field for the preparation of similar substances or the new methods described below.

Intermediate thiocarbamides of formula **TM**, in which R¹, R², R⁴, (#), Y and Z have the values indicated above have the values indicated above, are prepared by analogy with the known reaction of amines with isothiocyanates. However, as a consequence of severe steric hindrance, the final alkylation of N,N'(,N')-di(tri)-substituted TM thiocarbamides does not proceed unambiguously. In the course of creating the present invention, selective methods have been found for the alkylation of weakly reactive thiocarbamides with such a structure, allowing the optical activity of the compounds to be retained.

The basis for the preparation of new compounds of formula I according to the invention is a method which is comprised in that thiocarbamides of formula **TM** 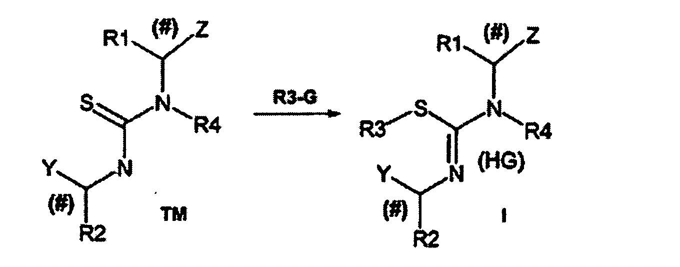 are reacted with an equivalent amount or an excess (up to 200%) of an appropriate alkylating agent with the formula R³-G (in which R³ has been defined above, and G is a leaving group such as halide, sulfate, alkylsulfonate, arylsulfonate, trifluoromethanesulfonate, alkylsulfate, perchlorate, acetate, and also optionally substituted benzoate), possibly in a polar inert solvent (such as alcohols, ethylene glycol, acetic acid, dimethyl-formamide, dimethylsulfoxide, sulfolane, acetone) at a temperature from room temperature to the boiling point of the reaction mixture.

According to the next aspect of the invention, methods are presented for the preparation of compounds of formula I, characterized in that the process is performed under the influence of SHF radiation in said solvents or in the form of a homogenized mixture of the starting compounds without a solvent. The structures of the compounds prepared were confirmed by chemical and spectral analysis data and other physico-chemical characteristics; the melting points were not corrected. PMR spectra were recorded using a Brucker CXP-200 instrument (200 MHz). The multiplet nature of the signals observed is indicated in abbreviated form: (s) - singlet, (d) - doublet, (t) - triplet, (q) - quadruplet and (m) multiplet.

The examples presented below illustrate, but do not limit, this invention.

### Example 1. S-Methyl-N-(S)-(-)-α-methylbenzyl-N'-(S)-(-)-α-methylbenzylisothiocarbamide hydriodide. (Compound 1).

A solution of 1.63 g (0.01 mole) of (S)-(-)-α-methylbenzylisothiocyanate and 1.21 g (0.01 mole) of (S)-(-)-α-methylbenzylamine in 50 ml of benzene was boiled for 4 hours. The solvent was evaporated off, and 1.7 g (0.012 mole) of methyl iodide in 50 ml of methanol were added to the resultant thiocarbamide. The reaction mixture was boiled for 1.5 hours, and the solvent and unreacted methyl iodide were evaporated off. Dry ether was poured over the resultant oil, and the crystals which precipitated were filtered off. Yield 2.1 g (49.2%), m.p. 86-88°C.

### Example 2. S-Methyl-N-(S)-(-)-α-methylbenzyl-N'-(4-methoxy)-benzyl-N'-(S)-(-)-α-methylbenzylisothiocarbamide hydriodide. (Compound 2).

A solution of 13.6 g (0.1 mole) of 4-methoxybenzaldehyde and 12.1 g (0.1 mole) of (S)-(-)-α-methylbenzylamine in 100 ml of toluene is boiled with a Dean and Stark fitting until the distillation of water has ceased. The reaction mixture is evaporated. The resultant imine is reduced by catalytic hydrogenation: a Schiff base (20 g) in 50 ml of isopropanol and 2 g of 1% Pd/C are placed in a unit for hydrogenation at atmospheric pressure. Reduction is continued until the absorption of hydrogen ceases. The reaction mixture is cooled, the catalyst is filtered off, and the solvent is evaporated off. The resultant oil is distilled, taking off the 168-172°C/1 mm Hg fraction. 1.63 g (0.01M) of (S)-(-)-α-methylbenzylisothiocyanate are added to 2.40 g (0.01 mole) of N-(4-methoxy)benzyl-N-(S)-(-)-α-methylbenzylamine in 50 ml of methanol, boiled for 1.5 hours, and 1.7 g (0.012 mole) of methyl iodide are added. The reaction mixture is boiled for 24 hours. The solvent is evaporated off, the resultant oil is crystallized from dry ether, and the crystals are filtered off and recrystallized from 20 ml of isopropanol.
Yield 2.0 g (35.5%), m.p. 117-119°C.

### Example 3. S-Methyl-N-4-fluorobenzyl-N'-[1-S(-)-phenylpropyl]-N'-isobutylisothiocarbamide. (Compound 3).

A solution of 13.5 g (0.1M) of (S)-(-)-1-phenylpropyl-amine and 7.2 g (0.1M) of isobutyraldehyde in 100 ml of toluene is boiled with a Dean and Stark fitting until the distillation of water has ceased. The solvent is evaporated off. The resultant imine is reduced by catalytic hydrogenation: a Schiff base (20 g) in 50 ml of isopropanol and 2 g of 1% Pd/C are placed in a unit for hydrogenation at atmospheric pressure. Reduction is continued until the absorption of hydrogen ceases. The reaction mixture is cooled, the catalyst is filtered off, and the solvent is evaporated off. The resultant oil is distilled, taking off the 112-116°C/1 mm Hg fraction. 1.79 g (0.01M) of 4-fluorobenzylisothiocyanate are added to 2.09 g (0.01 mole) of N-[1-S(-)-phenylpropyl]-N-isobutylamine in 50 ml of methanol, boiled for 8 hours, and 1.7 g (0.012 mole) of methyl iodide are added. The reaction mixture is boiled for 4 hours. The solvent is evaporated off, the resultant oil is crystallized from dry ether, and the crystals are filtered off and recrystallized from 20 ml of isopropanol.
Yield 2.3 g (46.0%), m.p. 111-113°C.

### Example 4. S-Methyl-N-4-methoxybenzyl-N'-3,4-dimethoxy-phenetyl-N'-1-phenylethylisothiocarbamide hydriodide. (Compound 4).

A solution of 12.0 g (0.1M) of acetophenone and 18.1 g (0.1M) of 3,4-dimethoxyphenetylamine in 100 ml of toluene are boiled with a Dean and Stark fitting until the distillation of water has ceased. The solvent is evaporated off. The resultant imine is reduced by catalytic hydrogenation: a Schiff base (20 g) in 50 ml of isopropanol and 2 g of 1% Pd/C are placed in a unit for hydrogenation at atmospheric pressure. Reduction is continued until the absorption of hydrogen ceases. The reaction mixture is cooled, the catalyst is filtered off, and the solvent is evaporated off. The resultant oil is distilled, taking off the 184-188°C/1 mm Hg fraction. 1.79 g (0.01M) of 4-methoxybenzylisothiocyanate are added to 3.03 g (0.01 mole) of N-3,4-dimethoxyphenetyl-N-1-phenylethylamine in 50 ml of methanol, boiled for 8 hours and 1.7 g (0.012 mole) of methyl iodide are added. The reaction mixture is boiled for 4 hours. The solvent is evaporated off, the resultant oil is crystallized from dry ether, and the crystals are filtered off and recrystallized from 20 ml of isopropanol.
Yield 2.2 g (36.3%), m.p. 121-123°C.

### Example 5. S-Methyl-N-2-tetrahydrofurfuryl-N'-5-methylfurfuryl-N'-2-phenylethylisothiocarbamide hydriodide. (Compound 5).

A solution of 11.0 g (0.1M) of 5-methylfurfurol and 12.1 g (0.1M) of 2-phenylethylamine in 100 ml of toluene is boiled with a Dean and Stark fitting until the distillation of water has ceased. The solvent is evaporated off. The resultant imine is reduced by catalytic hydrogenation: a Schiff base (20 g) in 50 ml of isopropanol and 2 g of 1% Pd/C are placed in a unit for hydrogenation at atmospheric pressure. Reduction is continued until the absorption of hydrogen ceases. The reaction mixture is cooled, the catalyst is filtered off, and the solvent is evaporated off. The resultant oil is distilled, taking off the 132-137°C/1 mm Hg fraction. 1.43 g (0.01M) of tetrahydrofurfurylisothiocyanate are added to 2.33 g (0.01 mole) of N-5-methylfurfuryl-N-2-phenylethylamine in 50 ml of methanol and boiled for 8 hours, and 1.7 g (0.012 mole) of methyl iodide are added. The reaction mixture is boiled for 4 hours. The solvent is evaporated off, the resultant oil is crystallized from dry ether, and the crystals are filtered off and recrystallized from 20 ml of isopropanol.
Yield 1.7 g (34.0%), m.p. 105-107°C.

### Example 6. S-Methyl-N-piperonyl-N'-2-thienylmethyl-N'-isopropylisothiocarbamide hydriodide. (Compound 6).

A solution of 11.6 g (0.1M) of thiophene-2-carbaldehyde and 6.5 g (0.11M) of isopropylamine in 100 ml of toluene is boiled with a Dean and Stark fitting until the distillation of water has ceased. The solvent and unreacted amine are evaporated off. 2 g (0.06M) of sodium borohydride are added in small portions with stirring to a solution of the resultant imine in 100 ml of methanol. The reaction mixture is then boiled for 1 hour, the solvent is evaporated off, and the residue is dissolved in 200 ml of methylene chloride and is washed with water (3*100 ml). The organic phase is separated off, dried with potash and the solvent evaporated off. The resultant oil is distilled, taking off the 135-140°C/10 mm Hg fraction. 1.93 g (0.01M) of piperonylisothiocyanate are added to 1.75 g (0.01 mole) of N-2-thienyl-N-isopropylamine in 50 ml of methanol, boiled for 8 hours and 1.7 g (0.012 mole) of methyl iodide are added. The reaction mixture is boiled for 4 hours. The solvent is evaporated off, the resultant oil is crystallized from dry ether, and the crystals are filtered off and recrystallized from 20 ml of isopropanol.
Yield 2.1 g (42.9%), m.p. 91-93°C.

### Example 7. S-Methyl-N-[1-S(-)-phenylethyl]-N'-[1-S(-)-phenylethyl]-N'-isobutylisothiocarbamide hydriodide. (Compound 7).

A solution of 12.1 g (0.1M) of S-(-)-1-phenylethylamine and 7.2 g (0.1M) of isobutyraldehyde in 100 ml of toluene is boiled with a Dean and Stark fitting until the distillation of water has ceased. The solvent is evaporated off. The resultant imine is reduced by catalytic hydrogenation: a Schiff base (19 g) in 50 ml of isopropanol and 2 g of 1% Pd/C are placed in a unit for hydrogenation at atmospheric pressure. Reduction is continued until the absorption of hydrogen ceases. The reaction mixture is cooled, the catalyst is filtered off, and the solvent is evaporated off. The resultant oil is distilled, taking off the 152-155°C/10 mm Hg fraction. 1.49 g (0.01M) of 1-S(-)-phenylethylisothiocyanate are added to 1.95 g (0.01 mole) of N-[1-S(-)-phenylethyl]-N-isobutylamine in 50 ml of methanol, boiled for 8 hours, and 1.7 g (0.012 mole) of methyl iodide are added. The reaction mixture is boiled for 4 hours. The solvent is evaporated off, the resultant oil is crystallized from dry ether, and the crystals are filtered off and recrystallized from 20 ml of isopropanol.
Yield 1.8 g (47.3%), m.p. 105-107°C.

The following optically active isothiocarbamides were prepared by a similar method, starting from the corresponding chiral 1-phenylethylamines:

### S-Methyl-N-[1-S(-)-phenylethyl]-N'-[1-R(+)-phenylethyl]-N'-isobutylisothiocarbamide hydriodide. (Compound 8) .

Yield 1.8 g (47.3%), m.p. 107-109°C.

### S-Methyl-N-[1-R(+)-phenylethyl]-N'-[1-R(+)-phenylethyl]-N'-isobutylisothiocarbamide hydriodide. (Compound 9).

Yield 1.7 g (35.2%), m.p. 106-108°C.

### S-Methyl-N-[1-R(+)-phenylethyl]-N'-[1-S(-)-phenylethyl]-N'-isobutylisothiocarbamide hydriodide. (Compound 10).

Yield 1.7 g (35.2%), m.p. 105-107°C.

### Example 8. S-Methyl-N-benzyl-N'-benzylisothio-carbamide. (Compound 11).

0.3 g of sulfur and 4.6 g (0.06 mole) of carbon disulfide are added to a solution of 10.7 g (0.1 mole) of benzylamine in 150 ml of ethanol. The mixture is heated on a water bath with a reflux condenser until the evolution of hydrogen sulfide has completely ceased. The sulfur is filtered off, and part of the alcohol is evaporated off under reduced pressure. After cooling, the N-benzyl-N'-benzylthiocarbamide which has precipitated is filtered off and recrystallized from 100 ml of isopropanol. 1.7 g (0.012 mole) of methyl iodide are added to 2.56 g (0.01 mole) of the prepared thiocarbamide in 50 ml of acetone. The reaction mixture is held at a temperature of 50°C for 24 hours. The crystals which have precipitated are filtered off and recrystallized from 20 ml of isopropanol.
Yield 3.2 g (80.4%), m.p. 116-118°C.
Calculated: C 48.25%, H 4.81%, N 7.03%, C16H19IN2S. Found: C 48.44%, H 5.02%, N 7.39%.
PMR spectrum (DMSO-d6, ppm, δ): 2.90 (3H, s, SCH3), 4.75 (2H, d, CH2Ph), 4.90 (2H, d, CH2Ph), 7.05-7.55 (10H, m, Ph), 9.50 (1H, broad t, NH), 9.90 (1H, broad t, NH).

The following were prepared by a similar method from N-benzyl-N'-benzylthiocarbamide and the corresponding alkyl halide:

### S-Ethyl-N-benzyl-N'-benzylisothiocarbamide hydriodide (Compound 12).

Yield 3.0 g (72.8%), m.p. 86-88°C.
Calculated: C 49.52%, H 5.13%, N 6.78%, C17H21IN2S. Found: C 49.46%, H 5.42%, N 6.38%.
PMR spectrum (DMSO-d6, ppm, δ): 1.35 (3H, t, CH3), 3.45 (2H, q, SCH2), 4.70 (2H, d, CH2Ph), 4.90 (2H, d, CH2Ph), 7.10-7.55 (10H, m, Ph), 9.60 (1H, broad t, NH), 9.95 (1H, broad t, NH).

### S-Benzyl-N-benzyl-N'-benzylisothiocarbamide. (Compound 13).

Yield 3.3 g (77.3%), m.p. 145-147°C.
Calculated C 61.83%, H 5.42%, N 6.55%, C22H23BrN2S. Found: C 61.48%, H 5.47%, N 6.35%.
PMR spectrum (DMSO-d6, ppm, δ): 4.65 (2H, s, SCH2), 4.75 (2H, d. CH2Ph), 4.95 (2H, d, CH2Ph), 7.10-7.55 (15H, m, Ph), 9.80 (1H, broad t, NH), 10.10 (1H, broad t, NH).

### S-4-Nitrobenzyl-N-benzyl-N'-benzylisothiocarbamide hydrochloride. (Compound 14).

Yield 3.1 g (72.6%), m.p. 158-160°C.
Calculated: C 61.75%, H 5.18%, N 9.82%, C22H22C1N302S. Found: C 61.43%, H 5.27%, N 9.55%.
PMR spectrum (DMSO-d6, ppm, δ): 4.75 (2H, d, CH2Ph), 4.85 (2H, d, CH2Ph), 4.95 (2H, s, SCH2), 7.10-7.30 (10H, m, Ph), 7.40-7.90 (4H, dd, PhNO2), 10.60 (1H, broad t, NH), 10.80 (1H, broad t, NH).

### S-4-Carboxybenzyl-N-benzyl-N'-benzylisothiocarbamide hydrobromide. (Compound 15).

Yield 2.7 g (57.3%), m.p. 137-139°C.
Calculated: C 58.60%, H 4.92%, N 5.94%, C23H23BrN2O2S. Found: C 58.43%, H 5.07%, N 5.59%.
PMR spectrum (DMSO-d6, ppm, δ): 4.55 (2H, s, SCH2), 4.65 (2H, d, CH2Ph), 4.70 (2H, d, CH2Ph), 6.90-7.10 (10H, m, Ph), 7.20-7.70 (4H, dd, Ph-COOH), 10.00 (1H, broad t, NH), 10.10 (1H, broad t, NH).

### S-Allyl-N-benzyl-N'-benzylisothiocarbamide hydrobromide. (Compound 16).

Yield 2.5 g (66.3%), m.p. 81-83°C.
Calculated: C 57.29%, H 5.61%, N 7.42%, C18H21BrN2S. Found: C 57.48%, H 5.42%, N 7.35%.
PMR spectrum (DMSO-d6, ppm, δ): 4.65 (2H, d, SCH2), 4.75 (2H, d, CH2Ph), 4.85 (2H, d, CH2Ph), 5.10-5.40 (2H, m, =CH2), 5.75 (1H, m, =CH), 7.20-7.50 (10H, m, Ph), 9.80 (1H, broad t, NH), 10.10 (1H, broad t, NH).

### S-2-Cyclohexenyl-N-benzyl-N'-benzylisothiocarbamide hydrobromide. (Compound 17).

Yield 2.2 g (52.8%), m.p. 145-147°C.
Calculated: C 60.43%, H 6.04%, N 6.71%, C21H25BrN2S. Found: C 60.33%, H 5.91%, N 6.37%.
PMR spectrum (DMSO-d6, ppm, δ): 1.55-2.10 (6H, m, CH2CH2CH2), 4.75 (2H, d, CH2Ph), 4.85 (2H, d, CH2Ph), 4.90 (1H, m, SCH), 5.65 (1H, m, =CH), 6.05 (1H, m, =CH), 7.20-7.60 (10H, m, Ph), 10.00 (1H, broad t, NH), 10.10 (1H, broad t, NH).

### S-2-Phenylethyl-N-benzyl-N'-benzylisothiocarbamide hydrobromide. (Compound 18).

Yield 2.2 g (49.9%), m.p. 78-80°C.
Calculated: C 62.58%, H 5.71%, N 6.35%, C23H25BrN2S. Found: C 62.48%, H 5.46%, N 6.39%.
PMR spectrum (DMSO-d6, ppm, δ): 2.80 (2H, t, CH2Ph), 3.70 (2H, t, SCH2), 4.55 (2H, d, CH2Ph), 5.00 (2H, d, CH2Ph), 7.10-7.75 (15H, m, Ph), 10.00 (1H, broad t, NH), 10.30 (1H, broad t, NH).

### S-2-Ethoxycarbonyl-ethyl-N-benzyl-N'-benzylisothiocarbamide hydrobromide. (Compound 19).

Yield 1.8 g (41.2%), m.p. 95-97°C.
Calculated: C 54.92%, H 5.76%, N 6.40%, C20H25BrN202S. Found: C 54.61%, H 5.48%, N 6.31%.
PMR spectrum (DMSO-d6, ppm, δ): 1.45 (3H, t, CH3), 3.30 (2H, t, CH2C(O)), 3.60 (2H, q, OCH2), 3.70 (2H, t, SCH2), 4.75 (2H, d, CH2Ph), 4.90 (2H, d, CH2Ph), 7.10-7.55 (10H, m, Ph), 10.10 (1H, broad t, NH), 10.30 (1H, broad t, NH).

As noted above, compounds of formula I according to the invention possess the ability to potentiate the responses of AMPA/KA receptors, and also to block the responses of NMDA receptors, which is confirmed by the results of biological tests.

The authors employed two alternative approaches in a focused search for compounds able to potentiate the responses of AMPA/KA receptors and to elicit an improvement in memory and cognitive functions in behavioral experiments with animals.

The first was to determine the potentiating effect of new compounds on AMPA- and kainate-induced transmembrane currents in neurons of the Purkinje cerebellum of rats, and also blockade of NMDA-induced currents in the neurons of the cerebral cortex of rats;

The second was to demonstrate in behavioral experiments improvement in memory and cognitive functions in rats with a deficit of cholinergic neurons caused by the action of the neurotoxin Af64A (and with impairment of memory and learning functions as a consequence of this), and also in intact rats.

Method for assessing properties of the compounds potentiating AMPA/KA-induced currents and blocking NMDA-induced currents.

The experiments were performed using the patch-clamp method on freshly-isolated Purkinje neurons taken from the cerebellum of rats (aged 14-16 days). A modified method was used for isolation. Slices of cerebellum 400-600 µm in thickness were placed in a thermostat-controlled chamber with a volume of 10 ml. The solution for isolation had the following composition (in mM): NaCl 150.0, KCl 5.0, CaCl₂ 2.0, MgSO₄×7H₂O 2.0, HEPES 10.0, glucose 15.0, pH 7.42. The slices were incubated in this solution for 60 minutes, after which this solution was replaced by a similar solution containing pronase (2 mg/ml) and collagenase (1 mg/ml) and incubation was continued for 70 minutes. After washing with the original solution for 20 minutes, the slices were placed in a Petri dish and dissociated mechanically with the aid of a Pasteur pipette. The solutions were continuously bubbled with 100% O₂ at t° 34°C. The Purkinje neurons were placed in a working chamber with a volume of 0.6 ml. The working solution has the composition (in mM): NaCl 150.0, KCl 5.0, CaCl₂ 2.6, MgSO₄×7H₂O 2.0, HEPES 10.0, glucose 15.0, pH 7.36.

Slices of the cerebral cortex of rats were prepared and treated in a similar manner, with the difference that the age of the rats was 7-8 days and the incubation time with enzymes was 14 minutes.

Transmembrane currents were induced by activation of AMPA/KA receptors by applying solutions of kainic acid, the agonist of these receptors, and by activation of NMDA receptors by applying solutions of N-methyl-D-aspartic acid (NMDA), the agonist of these receptors, using the rapid superfusion method. Currents were recorded with the aid of borosilicate microelectrodes (resistance 1.5-2.5 mohm) filled with the following composition (in mM): KCl 100.0, EGTA 11.0, CaCl₂ 1.0, MgCl₂ 1.0, HEPES 10.0, ATP 5.0, pH 7.2.

An EPC9 (HEKA, Germany) was used for recording. Currents were recorded on a Pentium-100 PC hard disc using the Pulse program, also purchased from the HEKA company. The results were processed with the aid of the Pulsefit program (HEKA).

The application of kainate induces inward transmembrane currents in the Purkinje neurons. Addition of the compounds to the perfusion solution elicits an increase in the amplitude of the currents. This increase depends on the compound, on its concentration, and on the time which has passed since the start of application of the substance.

The application of NMDA induces inward currents in cerebral cortex neurons. Addition of the compounds to the perfusion solution elicits a decrease in the amplitude.

**Example.** Compound **18.** In a dose of 20 µM, compound **18** elicits an 8-12% (average 10%) increase in kainate-induced currents. Washing out for 3-5 minutes returns the amplitude of the responses to the control value.

In cerebral cortex neurons, compound **18** elicits a decrease in the NMDA-induced currents. By 25-35% (average 30%) in a concentration of 3 µM, and by 100% in a dose of 10 µM. In other words, it virtually completely blocks these currents. Washing out compound **18** for 3-6 minutes returns the amplitude of the NMDA-induced currents to the control value.

The results obtained are presented in table 1. The compounds are superior in activity to the standard substance Tacrine by a factor of 1.0-2.0. At the same time, they have no marked neurotoxic effect in the active test dose range, which makes them valuable for use in medicine, in the treatment and prevention of diseases in which the cholinergic neuromediator system is involved in the pathogenetic mechanism, and particularly in the treatment of neurodegenerative diseases such as Alzheimer's disease.

**Table 1**

| Activity of the compounds in potentiation of AMPA/KA induced responses in Purkinje neurons of the rat cerebellum and blockade of NMDA receptors in neurons of the cerebral cortex. | | |
|---|---|---|
| Compound No. | Potentiation of kainate-induced currents (%) | Blockade of NMDA-induced currents (%) |
| **17** | 60-100% at 0.5-1.0 µM | 30-50% at 0.5 µM 100% at 2 µM |
| | 0% at 10 µM | |
| **13** | 0 at 0.5-30 µM | 0 at 0.5-30 µM |
| **16** | 0 at 0.5-30 µM | 0 at 0.5-30 µM |
| | | |
| **11** | 0 at 0.5-30 µM | 0 at 0.5-30 µM |
| **15** | 0 at 0.5-30 µM | 0 at 0.5-30 µM |
| **14** | 0 at 0.5-30 µM | 0 at 0.5-30 µM |
| **19** | 0 at 0.5-30 µM | 0 at 0.5-30 µM |
| **12** | 0 at 0.5-30 µM | 0 at 0.5-30 µM |
| **18** | 10% at 20 µM | 30% at 3 µM, |
| | | 100% at 10 µM |
| **Tacrine** | 0 at 0.5-30 µM | 0 at 0.5-30 µM |
| **Memantine** | 15% at 30 µM | 50% at 17 µM |
| ***Cyclothiazide*** | 20% at 1 µM | 0 at 1-30 µM |

The next aspect of the invention is compounds of general formula I, which possess the property of potentiating (positively modulating) glutamate AMPA/KA receptors, while at the same time blocking transmembrane currents induced by the activation of glutamate NMDA receptors.

The next aspect of the invention is a method of studying the glutamatergic system, which comprises the introduction of specific pharmacological markers as molecular tools, compounds of general formula I being used as such markers in connection with their ability to potentiate AMPA/KA receptors and also to block NMDA receptors.

The next aspect of the invention is a method for the treatment and prevention of neurological disorders and diseases associated with dysfunction of glutamatergic neurotransmission by acting on AMPA/KA and NMDA receptors by the administration of an effective amount of a compound of general formula I.

The next aspect of the invention is a method for substantial enhancement of memory and activation of the learning process by positive modulation of AMPA/KA receptors by the administration of an effective amount of a compound of general formula I.

The dose of active component (a compound of formula I or its pharmaceutically acceptable salts) prescribed varies in dependence on many factors, such as the age, sex and weight of the patient, the symptoms and severity of the disease, the specific compound prescribed, the method of administration, and the form of the preparation in which the active compound is prescribed.

The total prescribed dose is normally from 1 to 200 mg per day. The total dose may be divided into several doses, to be taken, for example, from 1 to 4 times per day. With oral administration, the range of total doses of active substance is from 10 to 200 mg, preferably from 15 to 150 mg, per day. With parenteral administration, the prescribed dose range is from 5 to 100 mg, preferably from 5 to 50 mg, per day, while for intravenous injections it is from 1 to 50 mg, preferably from 1 to 25 mg, per day. The precise dose can be selected by the attending physician.

As is normally the case in medicine, it is recommended that compounds of formula I according to the present invention be taken in the form of compositions, which constitute respectively the next aspect of the invention.

A pharmaceutical composition according to the invention is prepared using the procedures generally employed in this field and includes a pharmacologically effective amount of the active agent, which comprises a compound of formula I or its pharmaceutically acceptable salt (hereinafter referred to as "the active compound"), normally constituting from 5 to 30 wt.%, in combination with one or more pharmaceutically acceptable excipients, such as fillers, binders, disintegrating agents, adsorbents, aromatizers and flavorings. In accordance with known methods, pharmaceutical compositions may be presented in various liquid or solid forms.

Examples of solid medicinal forms include, for example, tablets, pills, gelatin capsules, etc.

Examples of liquid medicinal forms for injection and parenteral administration include solutions, emulsions, suspensions, etc.

Compositions are, as a rule, prepared using standard procedures, which provide for mixing of the active compound with a liquid or finely ground solid carrier.

Compositions according to the invention in the form of tablets contain from 5 to 30% of the active compound and a filler (fillers) or carrier (carriers). The following are used as such for tablets: **a) fillers:** beet sugar, lactose, glucose, sodium chloride, sorbitol, mannitol, glycol, dicalcium phosphate; **b) binders**: magnesium aluminum silicate, starch paste, gelatin, tragacanth, methylcellulose, carboxymethylcellulose and polyvinylpyrrolidone; **c) disintegrating agents:** dextrose, agar, alginic acid or its salts, starch, Tween.

### EXAMPLE 1.

100 mg tablets, each containing 15.0 mg of compound **17**

| | |
|---|---|
| Compound **17** | 15.0 mg |
| Lactose | 40.0 mg |
| Alginic acid | 20.0 mg |
| Citric acid | 5.0 mg |
| Tragacanth | 20.0 mg |

A tablet may be made by compressing or molding the active ingredient with one or more excipients.

Compressed tablets are made in a special unit. The active ingredient in the free form, such as a powder or granules, in an amount of 150 g (the amount of substance needed for the production of 10000 tablets) is mixed with the tragacanth binder (200 g), mixed with the lactose filler (400 g), and the alginic acid disintegrating agent (200 g) and citric acid odorizer (50 g) are added to the mixture.

Colorants and stabilizers are employed in addition for gelatin capsules. Tetrazine and indigo are used as the colorants, while the stabilizers may for example be sodium metabisulfite and sodium benzoate. The gelatin capsules now proposed contain from 1 to 20% of the active ingredient.

### EXAMPLE 2.

500 mg capsules, each containing 50 mg of compound **18**

| | |
|---|---|
| Compound **18** | 50.0 mg |
| Glycerine | 100.0 mg |
| Sugar syrup | 290.0 mg |
| Peppermint oil | 40.0 mg |
| Sodium benzoate | 10.0 mg |
| Ascorbic acid | 5.0 mg |
| Tetrazine | 5.0 mg |

500 g of the active substance (compound 18) (the amount needed for the preparation of 10000 capsules) is finely ground and mixed in a mixer with glycerine (1000 g) and sugar syrup (2900 mg). After mixing, peppermint oil (400 g), sodium benzoate (100 g), ascorbic acid (50 g) and tetrazine (50 g) are added to the mixture. Gelatine capsules are prepared by the droplet method. This method permits the simultaneous dropwise metering of drug solution and heated gelatin mass (900 g of gelatin) into cooled vaseline oil. It results in the formation of seamless spherical gelatin capsules filled with the drug mixture, fully ready for use and containing 50 mg of active substance.

Injectable forms of a composition preferably comprise isotonic solutions or suspensions. The above-mentioned forms may be sterilized and contain additives such as **preservatives:** sodium metabisulfite, benzoic acid, sodium benzoate, or a mixture of methylparaben and propylparaben; **stabilizers:** apricot gum, gum arabic, dextrin, starch paste, methylcellulose, Tween; **salts** regulating osmotic pressure (sodium chloride), or **buffers**. In addition, they may contain other therapeutically useful substances.

### EXAMPLE 3.

2 ml ampoules, each containing 20 mg of compound **17**

| | |
|---|---|
| Compound **17** | 20.0 mg |
| Sodium chloride 0.9% solution | 1.6 ml |
| Benzoic acid | 10.0 mg |
| Methylcellulose | 10.0 mg |
| Peppermint oil | 0.4 ml |

For the preparation of injectable forms, active compound **17** (20 g; the amount needed for the preparation of 1000 ampoules) is finely ground and mixed in a mixer with peppermint oil (400 ml), then methylcellulose (10 g) is added, mixed with 0.9% sodium chloride solution (1600 ml), and benzoic acid (10 g) is added. The resultant solution is packaged into 2 ml ampoules and sterilized with steam for 30 min.

## Claims

**1. S-substituted N-1-[(hetero)aryl]alkyl-N'-1-[(hetero)aryl]alkylisothiocarbamides of general formula I:** 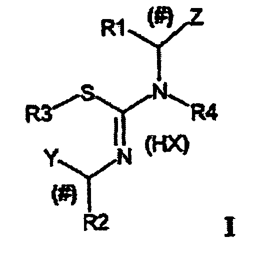 in which:
Y and Z may be the same or different and are, independently, possibly substituted aryl or possibly substituted (possibly partially or fully hydrogenated) heteroaryl;
the symbol (#) signifies the possibility of the presence of a chiral carbon atom;
R¹ and R² may be the same or different and are, independently, H or lower alkyl;
R³ is lower alkyl, possibly substituted alkenyl, possibly substituted cycloalkyl, cycloalkenyl, aralkyl or a group with the general formula:
-(CH₂)ₙ-W
in which: n = 1-4, while W is selected from a group consisting of:
possibly substituted aryl;
possibly substituted (possibly partially or fully hydrogenated) heteroaryl;
possibly substituted cycloalkyl;
ethenyl, optionally mono- or di-substituted with lower alkyl;
a COOR⁵ group (in which R⁵ is H, lower alkyl, phenyl);
a CH₂OR⁵ group (in which R⁵ has the values specified above);
a NR⁶R⁷ group (in which R⁶ and R⁷ may be the same or different, and each is, independently:
H; alkyl; cycloalkyl; aralkyl;
one of the substituents R⁶ or R⁷ may be an acyl group C(O)R⁵, in which R⁵ has the values specified above;
or in which R⁶ and R⁷ may together form an optionally substituted 1,4-butylene or 1,5-pentamethylene chain, and also a -CH₂CH₂-O-CH₂CH₂-chain);
a N (R⁸)₃⁺X⁻ group (in which R⁸ is lower alkyl, and X is the anion of a pharmacologically acceptable acid)
an N-phthalimido group, possibly substituted in the benzene ring.
R⁴ is H, lower alkyl, possibly substituted aryl, phenylethyl, possibly substituted in the phenyl ring, [(hetero)aryl]methyl or 1-[(hetero)aryl]ethyl;
**where:**
i) **R**^{**4**} **may have the values indicated only when R**^{**2**} **is not H;**
ii) when R² is H, R⁴ is H, lower alkyl, possibly substituted aryl or 2-phenylethyl, possibly substituted in the phenyl ring.
In the form of bases or salts of these with pharmacologically acceptable acids HX in the form of a racemic mixture or a mixture of stereoisomers.

**2.** The compounds as claimed in claim 1, comprising **S-substituted N-[(hetero)aryl]-methyl-N'-1-[(hetero)-aryl]alkylisothiocarbamides** with the general formula: 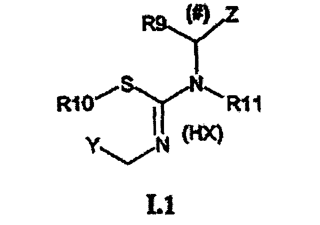 in which:
Y and Z have the values specified above for formula I; the symbol (#) signifies the possibility of the presence of a chiral carbon atom;
R⁹ is H or lower alkyl;
R¹⁰ is lower alkyl, alkenyl, aralkyl or a group with the general formula:
- (CH₂)ₘ-V
in which: m = 1-2, and V is selected from the group consisting of:
**(where m = 1)**
possibly substituted aryl;
possibly substituted cycloalkyl;
ethenyl, optionally monosubstituted with lower alkyl;
a COOR⁵ group (in which R⁵ has the values specified above);
a CH₂OR⁵ group (in which R⁵ has the values specified above);
a diphynylphosphinyl group;
**(where m = 2)**
aryl;
a COOR⁵ group (in which R⁵ has the values specified above);
N-piperidine;
N-morpholine;
N-pyrrolidine;
an N-phthalimido group, possibly substituted in the benzene ring.
R¹¹ is H, lower alkyl or 2-phenylethyl, possibly substituted in the phenyl ring.

**3.** The compounds as claimed in claim 2, comprising the derivatives **S-substituted N-[(hetero)aryl]methyl-N'-1-(R,S)-phenylethylisothiocarbamides** in the form of a racemic mixture (or an additive mixture of stereoisomers) of general formula **I.1.1:** 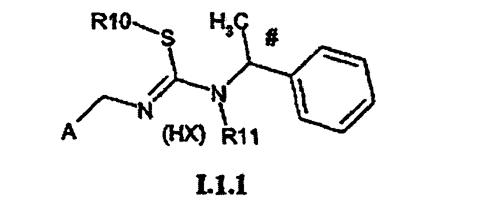 in which:
A is aryl, and also a heterocyclic substituent, namely:
optionally substituted 2- or 3-furyl,
optionally substituted 2- or 3-thienyl,
optionally substituted 2-pyrrolyl,
optionally substituted 3-indolyl;
optionally substituted 2-tetrahydrofurfuryl.
R¹⁰ has the values specified above for formula I.1;
R¹¹ has the values specified above for formula I.1;

**4.** Compounds as claimed in claim 1, comprising **S-substituted N-[(hetero)aryl]-methyl-N'-[(hetero)aryl]-methylisothiocarbamides** of general formula **I.2:** 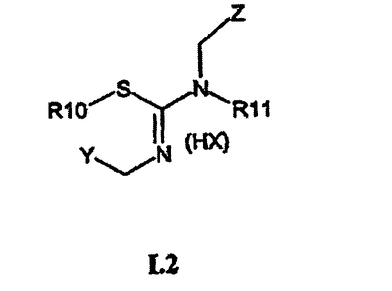 in which:
Y and Z have the values specified above for formula I;
R¹⁰ has the values specified above for formula I.1;
R¹¹ has the values specified above for formula I.1;

**5.** The compounds as claimed in claim 4, comprising **S-substituted N-[(hetero)aryl]methyl-N'-(heteroaryl)-methylisothiocarbamides** of general formula **I.2.1;** 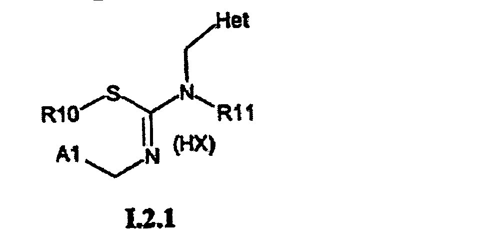 in which:
Het is:
optionally substituted 2- or 3-furyl;
optionally substituted 2- or 3-thienyl;
optionally substituted 2-pyrrolyl;
optionally substituted 3-indolyl;
optionally substituted 2-tetrahydrofurfuryl.
A¹ is:
phenyl, (optionally mono- or di-substituted with such substituents as halogen, lower alkyl, methoxy, ethoxy, trifluoromethyl);
methylenedioxyphenyl;
2-furyl;
2-tetrahydrofurfuryl.
R¹⁰ has the values specified above for formula I.1;
R¹¹ has the values specified above for formula I.1;

**6.** The compounds as claimed in claim 3, comprising **S-substituted N-[(hetero)aryl]methyl-N'-[1-R(+)-phenylethyl]isothiocarbamides** of general formula **I.1.1.1:** 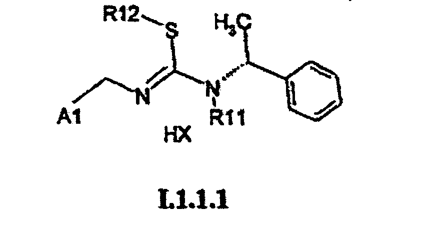 in which:
A¹ has the values specified above for formula I.2.1.
R¹¹ has the values specified above for formula I.1;
R¹² is selected from the group consisting of:
lower alkyl;
allyl;
cyclohexenyl;
benzyl, optionally substituted in the benzene ring;
2-phenylethyl;
2-(ethoxycarbonyl)ethyl;
2-N-phthalimidoethyl.

**7.** The compounds as claimed in claim 3, comprising **S-substituted N-[(hetero)aryl]-methyl-N'-[1-S(-)-phenyl-ethyl]isothiocarbamides** of general formula **I.1.1.2:** 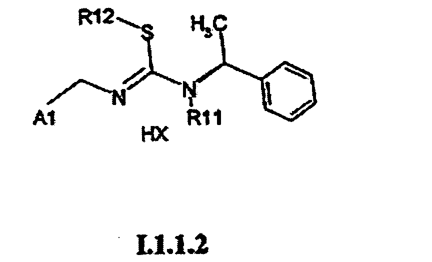 in which:
A¹ has the values specified above for formula I.2.1.
R¹¹ has the values specified above for formula I.1;
R¹² has the values specified above for formula I.1.1.1

**8.** The compounds as claimed in claim 1, comprising **S,N'-substituted N-[1-S(-)-phenylethyl]-N'-[(1-S(-)-phenyl-ethyl]isothiocarbamides** of general formula **I.3.a:** 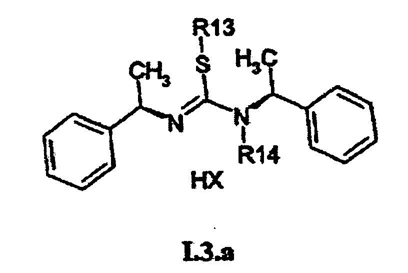 in which:
R¹³ is selected from the group consisting of:
lower alkyl;
allyl;
cyclohexenyl;
benzyl, optionally substituted in the benzene ring;
2-(phenyl)ethyl;
R¹⁴ is lower alkyl, optionally substituted benzyl, (heteroaryl)methyl or 2-phenylethyl;

**9.** The compounds as claimed in claim 1, comprising **S,N'-substituted N-[1-S(-)-phenylethyl]-N'**-**[1-(R(+)-phenyl-ethyl]isothiocarbamides** of general formula **I.3.b:** 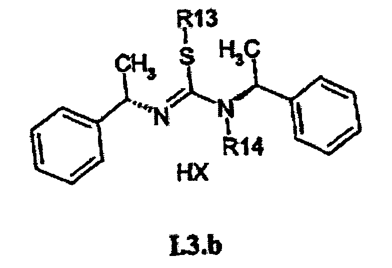 in which:
R¹³ and R¹⁴ have the values specified above for formula 1.3a;

**10.** The compounds as claimed in claim 1, comprising **S,N'-substituted N-[1-R(+)-phenylethyl]-N'-[1-(S(-)-phenyl-ethyl]isothiocarbamides** of general formula **I.3.c:** 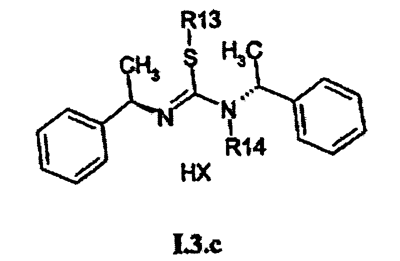 in which:
R¹³ and R¹⁴ have the values specified above for formula I.3a;

**11.** Compounds as claimed in claim 1, comprising **S,N'-substituted N-[1-R(+)-phenylethyl]-N'-[1-(R(+)-phenylethyl]isothiocarbamides** of general formula **I.3.d:** 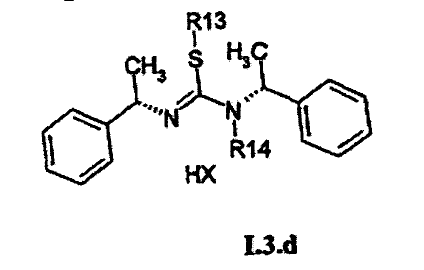 in which:
R¹³ and R¹⁴ have the values specified above for formula I.3a;

**12.** The compounds as claimed in claim 1, comprising S-methyl-N-1-S(-)-phenylethyl-N'-1-S(-)phenylethylisothiocarbamide and S-methyl-N-1-S(-)-phenylethyl-N'-(4-methoxy)benzyl-N'-1-S(-)-phenylethylisothiocarbamide in the form of the base or in the form of pharmacologically acceptable salts with HX.

**13.** The compound as claimed in claim 2, comprising S-methyl-N-4-fluorobenzyl-N'-[1-S(-)-phenylpropyl]-N'-isobutylisothiocarbamide and S-methyl-N-4-fluorobenzyl-N'-[1-R(+)-phenylpropyl]-N'-isobutylisothiocarbamide in the form of the base or in the form of pharmacologically acceptable salts with HX.

**13.** The compounds as claimed in claim 3, comprising S-methyl-N-4-methoxybenzyl-N'-3,4-dimethoxyphenetyl-N'-1-phenylethylisothiocarbamide in the form of the base or in the form of pharmacologically acceptable salts with HX.

**14.** The compounds as claimed in claim 4, comprising S-methyl-N-benzyl-N'-benzylisothiocarbamide, S-ethyl-N-benzyl-N'-benzylisothiocarbamide, S-benzyl-N-benzyl-N'-benzylisothiocarbamide, S-4-nitrobenzyl-N-benzyl-N'-benzylisothiocarbamide, S-4-carboxybenzyl-N-benzyl-N'-benzylisothiocarbamide, S-allyl-N-benzyl-N'-benzylisothiocarbamide, S-2-cyclohexenyl-N-benzyl-N'-benzylisothiocarbamide, S-2-phenylethyl-N-benzyl-N'-benzylisothiocarbamide, S-2-ethoxycarbonyl-ethyl-N-benzyl-N'-benzylisothiocarbamide in the form of the base or in the form of pharmacologically acceptable salts with HX.

**15.** The compounds as claimed in claim 5, comprising S-methyl-N-2-tetrahydrofurfuryl-N'-5-methylfurfuryl-N'-2-phenylethylisothiocarbamide and S-methyl-N-piperonyl-N'-2-thienylmethyl-N'-isopropylisothiocarbamide in the form of the base or in the form of pharmacologically acceptable salts with HX.

**16.** The compounds as claimed in claim 6, comprising S-methyl-N-benzyl-N'-1-R(+)-phenylethyl-N'-(cyclohex-3-enyl)methylisothiocarbamide and S-methyl-N-benzyl-N'-1-R(+)-phenylethyl-N'-2-phenylethylisothiocarbamide in the form of the base or in the form of pharmacologically acceptable salts with HX.

**17.** The compounds as claimed in claim 7, comprising S-methyl-N-benzyl-N'-1-S(-)-phenylethyl-N'-(cyclohex-3-enyl)methylisothiocarbamide and S-methyl-N-benzyl-N'-1-S(-)-phenylethyl-N'-2-phenylethylisothiocarbamide in the form of the base or in the form of pharmacologically acceptable salts with HX.

**18.** The compound as claimed in claim 8, comprising S-methyl-N-[1-S(-)-phenylethyl]-N'-[1-S(-)-phenylethyl]-N'-isobutylisothiocarbamide in the form of the base or in the form of pharmacologically acceptable salts with HX.

**19.** The compound as claimed in claim 9, comprising S-methyl-N-[1-S(-)-phenylethyl]-N'-[1-R(+)-phenylethyl]-N'-isobutylisothiocarbamide in the form of the base or in the form of pharmacologically acceptable salts with HX.

**20.** The compound as claimed in claim 10, comprising S-methyl-N-[1-R(+)-phenylethyl]-N'-[1-S(-)-phenylethyl]-N'-isobutylisothiocarbamide in the form of the base or in the form of pharmacologically acceptable salts with HX.

**21.** The compound as claimed in claim 11, comprising S-methyl-N-[1-R(+)-phenylethyl]-N'-[1-R(+)-phenylethyl]-N'-isobutylisothiocarbamide in the form of the base or in the form of pharmacologically acceptable salts with HX.

**22.** A method of preparing salts of compounds of formula I, which is comprised in that thiocarbamides of formula TM 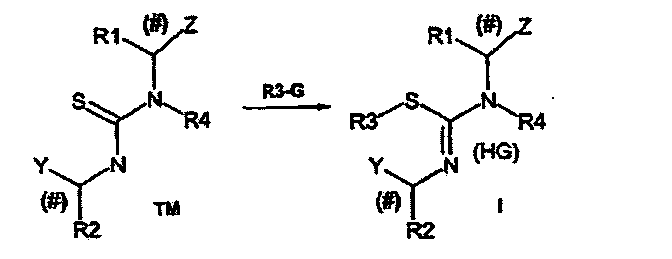 in which R¹, R², R⁴, (#), Y and Z have the values indicated above, are reacted with an equivalent amount or an excess (up to 200%) of an appropriate alkylating agent with the formula R³-G (in which R³ has been defined above, and G is a leaving group such as halide, sulfate, alkylsulfonate, arylsulfonate, trifluoromethanesulfonate, alkylsulfate, perchlorate, acetate, and also optionally substituted benzoate), possibly in a polar inert solvent (such as alcohols, ethylene glycol, acetic acid, dimethyl-formamide, dimethylsulfoxide, sulfolane, acetone) at a temperature from room temperature to the boiling point of the reaction mixture.

**23.** The method as claimed in claim 22, **characterized in that** the process is performed under the influence of SHF radiation in said solvents or in the form of a homogenized mixture of the starting compounds without a solvent.

**24.** The compounds of general formula I as claimed in any of claims 1-21, possessing the property of potentiating (positively modulating) glutamate AMPA/KA receptors, and at the same time blocking transmembrane currents induced by activation of glutamate NMDA receptors.

**25.** A method of studying the glutamatergic system, which comprises the introduction of specific pharmacological markers as molecular tools, compounds of general formula I as claimed in any of claims 1-21 being used as such markers in connection with their ability to potentiate AMPA/KA receptors and also to block NMDA receptors.

**26.** The compounds of general formula **I** as claimed in claim 24, capable of potentiating (positively modulating) glutamate AMPA/KA receptors and blocking NMDA receptors, and consequently possessing the properties of stimulators of cognitive functions and specific neuroprotectors.

**27.** The compounds of general formula **I** as claimed in any of claims 1-21, intended for the treatment and prevention of neurodegenerative diseases associated with dysfunction of glutamatergic neurotransmission.

**28.** The compounds of formula **I** as claimed in any of claims 1-21, intended for substantial enhancement of memory and activation of the learning process, i.e. as cognitive stimulators.

**29.** The compounds of general formula I as claimed in any of claims 1-21, as the active substance for the production of pharmaceutical compositions intended for the treatment and prevention of neurodegenerative diseases associated with dysfunction of glutamatergic neurotransmission, and also as cognitive stimulators.

**30.** A pharmaceutical composition containing a compound of general formula **I** as claimed in any of claims 1-21, intended for the treatment and prevention of neurodegenerative diseases associated with dysfunction of glutamatergic neurotransmission, and also as cognitive stimulators.

**31.** A method for the treatment and prevention of neurological disorders and diseases associated with dysfunction of glutamatergic neurotransmission by acting on AMPA/KA and NMDA receptors by the administration of an effective amount of a compound of general formula **I** as claimed in any of claims 1-21.

**32.** A method for substantial enhancement of memory and activation of the learning process by positive modulation of AMPA/KA receptors by the administration of an effective amount of a compound of general formula I as claimed in any of claims 1-21.
